# EUROPEAN PATENT SPECIFICATION

(11) **EP 1 366 063 B1**
(45) Date of publication and mention of the grant of the patent: **18.02.2009**
(21) Application number: 01990038.0
(22) Date of filing: 11.12.2001
(51) Int. Cl.: C07K 7/06, A61K 38/08

(54) **INHIBITORS OF LYN-ASSOCIATED SIGNAL TRANSDUCTION (LAST) AND THE USE THEREOF IN THE TREATMENT OF PROSTATE CANCER**
INHIBITOREN VON LYN-VERBUNDENER SIGNALTRANSDUKTION (LAST) UND DEREN VERWENDUNG ZUR BEHANDLUNG VON PROSTATAKREBS
INHIBITEURS DE LA TYROSINE KINASE LYN ET LEUR UTILISATION POUR LE TRAITEMENT DU CANCER DE LA PROSTATE

(30) Priority: 11.12.2000 US 735279
(43) Date of publication of application: 03.12.2003
(73) Proprietor: CHILDREN'S MEDICAL CENTER CORPORATION, Boston Massachusetts 02115 (US)
(72) Inventor: BEN-SASSON, Shmuel, 96555 Jerusalem (IL)
(74) Representative: Bergvall Eftring, Stina Lena
(86) International application number: PCT/US2001/047444
(87) International publication number: WO 2002/047710

(56) References cited:
- WO-A-98/53051
- US-A- 5 990 109
- AMOUI MEHRAN ET AL: "Src family-selective tyrosine kinase inhibitor, PP1, inhibits both Fc-epsilon-RI- and Thy-1-mediated activation of rat basophilic leukemia cells." EUROPEAN JOURNAL OF IMMUNOLOGY, vol. 27, no. 8, 1997, pages 1881-1886, XP001105330 ISSN: 0014-2980
- HE HONG ET AL: "An anti-Ras cancer potential of PP1, an inhibitor specific for Src family kinases: In vitro and in vivo studies." CANCER JOURNAL, vol. 6, no. 4, July 2000 (2000-07), pages 243-248, XP001105331 ISSN: 1528-9117
- BILLMORIA MALCOLM M ET AL: "Increasing Src kinase correlates with tumor aggressiveness in prostate cancer." PROCEEDINGS OF THE AMERICAN ASSOCIATION FOR CANCER RESEARCH ANNUAL, no. 41, March 2000 (2000-03), page 440 XP008008133 91st Annual Meeting of the American Association for Cancer Research.;San Francisco, California, USA; April 01-05, 2000, March, 2000 ISSN: 0197-016X

### SEQUENCE LISTING

<110> Ben-Sasson, Shmuel A.
<120> TREATMENT OF PROSTATE CANCER BY INHIBITING LYE TYROSINE KINASE
<130> 1242.1014-008
<140> US 09/735,279
   <141> 2000-12-11
<150> PCT/US98/10321
   <151> 1998-05-20
<150> US 08/861,153
   <151> 1997-05-21
<160> 82
<170> FastSEQ for Windows Version 4.0
<210> 1
   <211> 7
   <212> PRT
   <213> Artificial Sequence
<220>
   <223> Lyn
   Position 5 is D-lysine
<221> ACETYLATION
   <222> (1) ... (0)
<221> AMIDATION
   <222> (0) ... (7)
<400> 1
   Ile Val Thr Tyr Lys Lys Ile
    1               5
<210> 2
   <211> 8
   <212> PRT
   <213> Artificial Sequence
<220>
   <223> Lyn
<221> MYRISTATE
   <222> (1) ... (0)
<221> AMIDATION
   <222> (0) ... (8)
<400> 2
   Gly Ile Val Thr Tyr Gly Lys Ile
   1 5
<210> 3
   <211> 11
   <212> PRT
   <213> Artificial Sequence
<220>
   <223> Lyn
<221> MYRISTATE
   <222> (1)...(0)
<221> AMIDATION
   <222> (0)...(11).
<400> 3
   Gly Ile Val Thr Tyr Gly Lys Ile Pro Tyr Pro
   1 5 10
<210> 4
   <211> 6
   <212> PRT
   <213> Artificial Sequence
<220>
   <223> Lyn
<221> MYRISTATE
   <222> (1)...(0)
<221> AMIDATION
   <222> (0) ... (6)
<400> 4
   Gly Ile Val Thr Tyr Gly
   1 5
<210> 5
   <211> 9
   <212> PRT
   <213> Artificial Sequence
<220>
   <223> Lyn
<221> MYRISTATE
   <222> (1) ... (0)
   <223> Position 6 is D-isomer of lysine
<221> AMIDATION
   <222> (0) ... (9)
   <223> Position 9 is biotinylated lysine
<400> 5
   Gly Leu Val Thr Tyr Lys Lys Ile Lys
   1 5
<210> 6
   <211> 8
   <212> PRT
   <213> Artificial Sequence
<220>
   <223> Lyn
<221> MYRISTATE
   <222> (1)...(0)
<221> AMIDATION
   <222> (0)...(8)
<400> 6
   Gly Leu Val Thr Tyr Gly Lys Ile
   1 5
<210> 7
   <211> 8
   <212> PRT
   <213> Artificial Sequence
<220>
   <223> Lyn
<221> MYRISTATE
   <222> (1)...(0)
<221> AMIDATION
   <222> (0)...(8)
<400> 7
   Gly Ile Val Ser Tyr Gly Lys Ile
   1 5
<210> 8
   <211> 8
   <212> PRT
   <213> Artificial Sequence
<220>
   <223> Lyn
<221> MYRISTATE
   <222> (1) ... (0)
<221> AMIDATION
   <222> (0) ... (8)
<400> 8
   Gly Ile Val Thr Tyr Ala Lys Ile
   1 5
<210> 9
   <211> 8
   <212> PRT
   <213> Artificial Sequence
<220>
   <223> Lyn
<221> MYRISTATE
   <222> (1)...(0)
<221> AMIDATION
   <222> (0)...(8)
<400> 9
   Gly Ile Val Thr Phe Gly Lys Ile
   1 5
<210> 10
   <211> 8
   <212> PRT
   <213> Artificial Sequence
<220>
   <223> Lyn
<221> MYRISTATE
   <222> (1)...(0)
<221> AMIDATION
   <222> (0) ... (8)
<400> 10
   Gly Ile Val Thr Tyr Gly Lys Val
   1 5
<210> 11
   <211> 8
   <212> PRT
   <213> Artificial Sequence
<220>
   <223> Lyn
<221> MYRISTATE
   <222> (1) ... (0)
<221> AMIDATION
   <222> (0) ... (8)
<221> VARIANT
   <222> 7
   <223> Xaa = Any Amino Acid
<221> VARIANT
   <222> 7
   <223> Xaa = Orn
<400> 11
   Gly Ile Val Thr Tyr Gly Xaa Ile
    1                  5
<210> 12
   <211> 8
   <212> PRT
   <213> Artificial Sequence
<220>
   <223> Lyn
<221> VARIANT
   <222> 7
   <223> Xaa = Any Amino Acid
<221> MYRISTATE
   <222> (1)...(0)
<221> AMIDATION
   <222> (0)...(8)
<221> VARIANT
   <222> 7
   <223> Xaa = Orn
<400> 12
   Gly Ile Val Ser Tyr Gly Xaa Ile
    1                  5
<210> 13
   <211> 8
   <212> PRT
   <213> Artificial Sequence
<220>
   <223> Lyn
<221> MYRISTATE
   <222> (1) ... (0)
<221> AMIDATION
   <222> (0) ... (8)
<400> 13
   Gly Val Val Thr Tyr Gly Lys Ile
   1 5
<210> 14
   <211> 8
   <212> PRT
   <213> Artificial Sequence
<220>
   <223> Lyn
<221> MYRISTATE
   <222> (1) ... (0)
<221> AMIDATION
   <222> (0)...(8)
<400> 14
   Gly Ile Ala Thr Tyr Gly Lys Ile
   1 5
<210> 15
   <211> 8
<212> PRT
   <213> Artificial Sequence
<220>
   <223> Lyn
<221> MYRISTATE
   <222> (1) ... (0)
<221> AMIDATION
   <222> (0) ... (8)
<400> 15
   Gly Ile Ile Thr Tyr Gly Lys Ile
   1 5
<210> 16
   <211> 8
   <212> PRT
   <213> Artificial Sequence
<220>
   <223> Lyn
<221> MYRISTATE
   <222> (1) ... (0)
<221> AMIDATION
   <222> (0) ... (8)
<400> 16
   Gly Ile Leu Thr Tyr Gly Lys Ile
   1 5
<210> 17
   <211> 8
   <212> PRT
   <213> Artificial Sequence
<220>
   <223> lyn
<221> MYRISTATE
   <222> (1)...(0)
   <223> Position 6 is D-isomer of aspartic acid
<221> AMIDATION
   <222> (0) ... (8)
<400> 17
   Gly Leu Val Thr Tyr Asp Lys Ile
   1 5
<210> 18
   <211> 8
   <212> PRT
   <213> Artificial Sequence
<220>
   <223> Lyn
<221> MYRISTATE
   <222> (1)...(0)
   <223> Position 6 is D-isomer of lysine
<221> AMIDATION
   <222> (0) ... (8)
<400> 18
   Gly Leu Val Thr Tyr Lys Lys Ile
   1 5
<210> 19
   <211> 8
   <212> PRT
   <213> Artificial Sequence
<220>
   <223> Lyn
<221> MYRISTATE
   <222> (1) ... (0)
   <223> Position 6 is D-isomer of Serine
<221> AMIDATION
   <222> (0) ... (8)
<400> 19
   Gly Leu Val Thr Tyr Ser Lys Ile
   1 5
<210> 20
   <211> 8
   <212> PRT
   <213> Artificial Sequence
<220>
   <223> Lyn
<221> MYRISTATE
   <222> (1)...(0)
   <223> Position 6 is D-isomer of Methionine
<221> AMIDATION
   <222> (0) ... (8)
<400> 20
   Gly Leu Val Thr Tyr Met Lys Ile
   1 5
<210> 21
   <211> 6
<212> PRT
   <213> Artificial Sequence
<220>
   <223> Lyn
<221> MYRISTATE
   <222> (1) ... (0)
   <223> Position 6 is D-isomer of Lysine
<221> AMIDATION
   <222> (0) ... (6)
<400> 21
   Gly Ile Val Thr Tyr Lys
   1 5
<210> 22
   <211> 8
   <212> PRT
   <213> Artificial Sequence
<220>
   <223> Lyn
<221> MYRISTATE
   <222> (I) ... (0)
   <223> Position 6 is D-isomer of Lysine
<221> AMIDATION
   <222> (0) ... (8)
<400> 22
   Gly Ile Val Thr Tyr Lys Lys Ile
   1 5
<210> 23
   <211> 8
   <212> PRT
   <213> Artificial Sequence
<220>
   <223> Lyn
<221> MYRISTATE
   <222> (1)...(0)
   <223> Position 5 is di-iodo tyrosine
<221> AMIDATION
   <222> (0) ... (8)
   <223> Position 6 is D-isomer of lysine
<400> 23
   Gly Leu Val Thr Tyr Lys Lys Ile
   1 5
<210> 24
   <211> 8
   <212> PRT
   <213> Artificial Sequence
<220>
   <223> Lyn
<221> MYRISTATE
   <222> (1) ... (0)
   <223> Position 5 is di-iodo tyrosine
<221> AMIDATION
   <222> (0) ... (8)
<400> 24
   Gly Ile Val Thr Tyr Gly Lys Ile
    1                  5
<210> 25
   <211> 8
   <212> PRT
   <213> Artificial Sequence
<220>
   <223> Lyn
<221> MYRISTATE
   <222> (1) ... (0)
   <223> Position 6 is D-isomer of lysine
<221> AMIDATION
   <222> (0) ... (8)
   <223> Position 7 is D-isomer of lysine
<400> 25
   Gly Leu Val Thr Tyr Lys Lys Ile
    1                  5
<210> 26
   <211> 8
   <212> PRT
   <213> Artificial Sequence
<220>
   <223> Lyn
   Position 5 is di-iodo tyrosine
<221> MYRISTATE
   <222> (1)...(0)
   <223> Position 6 is D-isomer of lysine
<221> AMIDATION
   <222> (0)...(8)
   <223> Position 7 is D-isomer of lysine
<400> 26
   Gly Leu Val Thr Tyr Lys Lys Ile
    1                  5
<210> 27
   <211> 8
   <212> PRT
   <213> Artificial Sequence
<220>
   <223> Lyn
<221> MYRISTATE
   <222> (1) ... (0)
   <223> Position 7 is D-isomer of lysine
<221> AMIDATION
   <222> (0) ... (8)
<400> 27
   Gly Ile Val Thr Tyr Gly Lys Ile
   1 5
<210> 28
   <211> 8
   <212> PRT
   <213> Artificial Sequence
<220>
   <223> Lyn
<221> MYRISTATE
   <222> (1) ... (0)
<221> AMIDATION
   <222> (0)...(8)
<400> 28
   Gly Leu Val Thr Tyr Lys Lys Ile
   1 5
<210> 29
   <211> 8
   <212> PRT
   <213> Artificial Sequence
<220>
   <223> Lyn
   Position 5 is di-iodo tyrosine
<221> MYRISTATE
   <222> (1)...(0)
   <223> Position 6 is D-isomer of lysine
<221> AMIDATION
   <222> (0) ... (8)
   <223> Position 7 is D-isomer of lysine
<400> 29
   Gly Leu Val Thr Tyr Lys Lys Ile
   1 5
<210> 30
   <211> 8
   <212> PRT
   <213> Artificial Sequence
<220>
   <223> Lyn
<221> MYRISTATE
   <222> (1) ... (0)
   <223> Position 6 is D-isomer of lysine
<400> 30
   Gly Leu Val Thr Tyr Lys Lys Ile
   1 5
<210> 31
   <211> 9
   <212> PRT
   <213> Artificial Sequence
<220>
   <223> Lyn
   Position 5 is di-iodo tyrosine
<221> MYRISTATE
   <222> (1) ... (0)
   <223> Position 6 is D-isomer of lysine
<221> AMIDATION
   <222> (0)...(9)
   <223> Position 7 is D-isomer of lysine
<400> 31
   Gly Leu Val Thr Tyr Lys Lys Ile Lys
   1 5
<210> 32
   <211> 9
   <212> PRT
   <213> Artificial Sequence
<220>
   <223> Lyn
   Position 5 is di-iodo tyrosine
<221> MYRISTATE
   <222> (1) ... (0)
   <223> Position 6 is D-isomer of lysine
<221> AMIDATION
   <222> (0) ... (9)
   <223> Position 7 is D-isomer of lysine
   Position 9 is biotinylated lysine
<400> 32
   Gly Leu Val Thr Tyr Lys Lys Ile Lys
   1 5
<210> 33
   <211> 9
   <212> PRT
   <213> Artificial Sequence
<220>
   <223> Lyn
   Position 5 is di-iodo tyrosine
<221> MYRISTATE
   <222> (1) ... (0)
   <223> Position 6 is D-isomer of lysine
   Position 7 is D-isomer of lysine'
<221> AMIDATION
   <222> (0) ... (9)
   <223> Position 9 is Benzylamide of lysine
<400> 33
   Gly Leu Val Thr Tyr Lys Lys Ile Lys
   1 5
<210> 34
   <211> 11
   <212> PRT
   <213> Artificial Sequence
<220>
   <223> Lyn
   Position 6 is D-isomer of lysine
<221> MYRISTATE
   <222> (1) ... (0)
   <223> Position 7 is D-isomer of lysine
<221> AMIDATION
   <222> (0)...(11)
<400> 34
   Gly Leu Val Thr Tyr Lys Lys Ile Pro Tyr Pro
   1 5 10
<210> 35
   <211> 11
   <212> PRT
   <213> Artificial Sequence
<220>
   <223> Lyn
   Position 5 is di-iodo tyrosine
<221> MYRISTATE
   <222> (1) ... (0)
   <223> Position 6 is D-isomer of lysine
<221> AMIDATION
   <222> (0)...(11)
   <223> Position 7 is D-isomer of lysine
<400> 35
   Gly Leu Val Thr Tyr Lys Lys Ile Pro Tyr Pro
   1               5                   10
<210> 36
   <211> 8
   <212> PRT
   <213> Artificial Sequence
<220>
   <223> Lyn
   Position 5 is di-iodo tyrosine
<221> MYRISTATE
   <222> (1)...(0)
   <223> Position 6 is D-isomer of lysine
<221> AMIDATION
   <222> (0)...(8)
   <223> Position 7 is D-isomer of lysine
<400> 36
   Gly Ile Ala Thr Tyr Lys Lys Ile
   1               5
<210> 37
   <211> 8
   <212> PRT
   <213> Artificial Sequence
<220>
   <223> Lyn
   Position 5 is di-iodo tyrosine
<221> MYRISTATE
   <222> (1)...(0)
   <223> Position 6 is D-isomer of lysine
<221> AMIDATION
   <222> (0) ... (8)
   <223> Position 7 is D-isomer of lysine
<400> 37
   Gly Leu Val Thr Tyr Asp Lys Ile
   1 5
<210> 38
   <211> 9
   <212> PRT
   <213> Artificial Sequence
<220>
   <223> Lyn
   Position 6 is D-isomer of lysine
<221> MYRISTATE
   <222> (1) ... (0)
   <223> Position 9 is benzylamide lysine
<221> AMIDATION
   <222> (0)...(9)
<400> 38
   Gly Ile Ala Thr Tyr Lys Lys Ile Lys
   1 5
<210> 39
   <211> 9
   <212> PRT
   <213> Artificial Sequence
<220>
   <223> Lyn
   Position 6 is D-isomer of lysine
<221> MYRISTATE
   <222> (1)...(0)
   <223> Position 9 is benzylamide lysine
<221> AMIDATION
   <222> (0) ... (9)
<400> 39
   Gly Leu Val Thr Tyr Lys Lys Ile Lys
   1 5
<210> 40
   <211> 9
   <212> PRT
   <213> Artificial Sequence
<220>
   <223> Lyn
   Position 1 is oleoyl-glycine
   Position 6 is D-isomer of lysine
<221> AMIDATION
   <222> (0) ... (9)
   <223> Position 9 is benzylamide lysine
<400> 40
   Gly Ile Ala Thr Tyr Lys Lys Ile Lys
   1 5
<210> 41
   <211> 9
   <212> PRT
   <213> Artificial Sequence
<220>
   <223> Lyn
   Position 1 is oleoyl-glycine
   Position 6 is D-isomer of lysine
<221> AMIDATION
   <222> (0)...(9)
   <223> Position 9 is benzylamide lysine
<400> 41
   Gly Leu Val Thr Tyr Lys Lys Ile Lys
   1 5
<210> 42
   <211> 9
   <212> PRT
   <213> Artificial Sequence
<220>
   <223> Lyn
   Position 6 is D-isomer of lysine
<221> MYRISTATE
   <222> (1) ... (0)
<221> AMIDATION
   <222> (0) ... (9)
   <223> Position 9 is biotinylated lysine
<400> 42
   Gly Leu Val Thr Tyr Lys Lys Ile Lys
   1 5
<210> 43
   <211> 9
   <212> PRT
   <213> Artificial Sequence
<220>
   <223> Lyn
   Position 1 is stearyl-glycine
   Position 6 is D-isomer of lysine
<221> AMIDATION
   <222> (0) ... (9)
   <223> Position 9 is biotinylated lysine
<400> 43
   Gly Leu Val Thr Tyr Lys Lys Ile Lys
    1               5
<210> 44
   <211> 9
   <212> PRT
   <213> Artificial Sequence
<220>
   <223> Lyn
   Position 1 is phenyl-glycine
   Position 6 is D-isomer of lysine
<221> AMIDATION
   <222> (0)...(9)
   <223> Position 9 is biotinylated lysine
<400> 44
   Gly Leu Val Thr Tyr Lys Lys Ile Lys
    1               5
<210> 45
   <211> 9
   <212> PRT
   <213> Artificial Sequence
<220>
   <223> Lyn
   Position 1 is adamantan- glycine
   Position 6 is D-isomer of lysine
<221> AMIDATION
   <222> (0) ... (9)
   <223> Position 9 is biotinylated lysine
<400> 45
   Gly Leu Val Thr Tyr Lys Lys Ile Lys
   1 5
<210> 46
   <211> 9
   <212> PRT
   <213> Artificial Sequence
<220>
   <223> Lyn
   Position 1 is naphthalene- glycine
   Position 6 is D-isomer of lysine
<221> AMIDATION
   <222> (0) ... (9)
   <223> Position 9 is biotinylated lysine
<400> 46
   Gly Leu Val Thr Tyr Lys Lys Ile Lys
   1 5
<210> 47
   <211> 9
   <212> PRT
   <213> Artificial Sequence
<220>
   <223> Lyn
   Position 1 is myristoleyl- glycine
   Position 6 is D-isomer of lysine
<221> AMIDATION
   <222> (0) ... (9)
   <223> Position 9 is biotinylated lysine
<400> 47
   Gly Leu Val Thr Tyr Lys Lys Ile Lys
   1 5
<210> 48
   <211> 9
   <212> PRT
   <213> Artificial Sequence
<220>
   <223> Lyn
   Position 1 is toluen-glycine
   Position 6 is D-isomer of lysine
<221> AMIDATION
   <222> (0) ... (9)
   <223> Position 9 is biotinylated lysine
<400> 48
   Gly Leu Val Thr Tyr Lys Lys Ile Lys
   1 5
<210> 49
   <211> 9
   <212> PRT
   <213> Artificial Sequence
<220>
   <223> Lyn
   Position 1 is biphenyl-glycine
   Position 6 is D-isomer of lysine
<221> AMIDATION
   <222> (0) ... (9)
   <223> Position 9 is biotinylated lysine
<400> 49
   Gly Leu Val Thr Tyr Lys Lys Ile Lys
   1 5
<210> 50
   <211> 9
   <212> PRT
   <213> Artificial Sequence
<220>
   <223> Lyn
   Position 1 is Cinnamoyl-glycine
   Position 6 is D-isomer of lysine
<221> AMIDATION
   <222> (0)...(9)
   <223> Position 9 is biotinylated lysine
<400> 50
   Gly Leu Val Thr Tyr Lys Lys Ile Lys
   1 5
<210> 51
   <211> 9
   <212> PRT
   <213> Artificial Sequence
<220>
   <223> Lyn
   Position 1 is nitrobenzoyl- glycine
   Position 6 is D-isomer of lysine
<221> AMIDATION
   <222> (0)...(9)
   <223> Position 9 is biotinylated lysine
<400> 51
   Gly Leu Val Thr Tyr Lys Lys Ile Lys
   1               5
<210> 52
   <211> 9
   <212> PRT
   <213> Artificial Sequence
<220>
   <223> Lyn
   Position 1 is toluoyl-glycine
   Position 6 is D-isomer of lysine
<221> AMIDATION
   <222> (0) ... (9)
   <223> Position 9 is biotinylated lysine
<400> 52
   Gly Leu Val Thr Tyr Lys Lys Ile Lys
   1               5
<210> 53
   <211> 9
   <212> PRT
   <213> Artificial Sequence
<220>
   <223> Lyn
   Position 1 is furoyl-glycine
   Position 6 is D-isomer of lysine
<221> AMIDATION
   <222> (0) ... (9)
   <223> Position 9 is biotinylated lysine
<400> 53
   Gly Leu Val Thr Tyr Lys Lys Ile Lys
   1               5
<210> 54
   <211> 9
   <212> PRT
   <213> Artificial Sequence
<220>
   <223> Lyn
   Position 1 is benzoyl-glycine
   Position 6 is D-isomer of lysine
<221> AMIDATION
   <222> (0) ... (9)
   <223> Position 9 is biotinylated lysine
<400> 54
   Gly Leu Val Thr Tyr Lys Lys Ile Lys
   1 5
<210> 55
   <211> 9
   <212> PRT
   <213> Artificial Sequence
<220>
   <223> Lyn
   Position 1 is oleoyl-glycine
   Position 6 is D-isomer of lysine
<221> AMIDATION
   <222> (0)...(9)
   <223> Position 9 is biotinylated lysine
<400> 55
   Gly Leu Val Thr Tyr Lys Lys Ile Lys
   1 5
<210> 56
   <211> 9
   <212> PRT
   <213> Artificial Sequence
<220>
   <223> Lyn
   Position 1 is cyclohexane- glycine
   Position 6 is D-isomer of lysine
<221> AMIDATION
   <222> (0)...(9)
   <223> Position 9 is biotinylated lysine
<400> 56
   Gly Leu Val Thr Tyr Lys Lys Ile Lys
   1 5
<210> 57
   <211> 9
   <212> PRT
   <213> Artificial Sequence
<220>
   <223> Lyn
   Position 1 is norbornane- glycine
   Position 6 is D-isomer of lysine
<221> AMIDATION
   <222> (0) ... (9)
   <223> Position 9 is biotinylated lysine
<400> 57
   Gly Leu Val Thr Tyr Lys Lys Ile Lys
   1 5
<210> 58
   <211> 9
   <212> PRT
   <213> Artificial Sequence
<220>
   <223> Lyn
   Position 1 is z-caproic-glycine
   Position 6 is D-isomer of lysine
<221> AMIDATION
   <222> (0) ... (9)
   <223> Position 9 is biotinylated lysine
<400> 58
   Gly Leu Val Thr Tyr Lys Lys Ile Lys
   1 5
<210> 59
   <211> 9
   <212> PRT
   <213> Artificial Sequence
<220>
   <223> Lyn
   Position 1 is oleyl-glycine
   Position 6 is D-isomer of lysine
<221> AMIDATION
   <222> (0) ... (9)
   <223> Position 9 is benzylamide lysine
<400> 59
   Gly Leu Val Thr Tyr Lys Lys Ile Lys
   1 5
<210> 60
   <211> 9
   <212> PRT
   <213> Artificial Sequence
<220>
   <223> Lyn
   Position 6 is D-isomer of lysine
<221> MYRISTATE
   <222> (1) ... (0)
<221> AMIDATION
   <222> (0)...(9)
   <223> Position 9 is benzylamide lysine
<400> 60
   Gly Leu Val Thr Tyr Lys Lys Ile Lys
    1               5
<210> 61
   <211> 9
   <212> PRT
   <213> Artificial Sequence
<220>
   <223> Lyn
   Position 5 is di-iodo tyrosine
<221> MYRISTATE
   <222> (1) ... (0)
   <223> Position 6 is D-isomer of lysine
<221> AMIDATION
   <222> (0)...(9)
   <223> Position 9 is benzylamide lysine
<400> 61
   Gly Leu Val Thr Tyr Lys Lys Ile Lys
    1               5
<210> 62
   <211> 9
   <212> PRT
   <213> Artificial Sequence
<220>
   <223> Lyn
   Position 1 is oleyl-glycine
   Position 6 is D-isomer of lysine
<221> AMIDATION
   <222> (0) ... (9)
   <223> Position 7 is D-isomer of lysine
<400> 62
   Gly Leu Val Thr Tyr Lys Lys Ile Lys
    1               5
<210> 63
   <211> 9
   <212> PRT
   <213> Artificial Sequence
<220>
   <223> Lyn
   Position 1 is ricinolelyl-glycine
   Position 6 is D-isomer of lysine
<221> AMIDATION
   <222> (0) ... (9)
   <223> Position 7 is D-isomer of lysine
<400> 63
   Gly Leu Val Thr Tyr Lys Lys Ile Lys
   1 5
<210> 64
   <211> 9
   <212> PRT
   <213> Artificial Sequence
<220>
   <223> Lyn
   Position 6 is D-isomer of lysine
<221> MYRISTATE
   <222> (1) ... (0)
<221> AMIDATION
   <222> (0) ... (9)
   <223> Position 7 is D-isomer of lysine
<400> 64
   Gly Leu Val Thr Tyr Lys Lys Ile Lys
   1 5
<210> 65
   <211> 9
   <212> PRT
   <213> Artificial Sequence
<220>
   <223> Lyn
   Position 5 is di-iodo tyrosine
<221> MYRISTATE
   <222> (1) ... (0)
   <223> Position 6 is D-isomer of lysine
<221> AMIDATION
   <222> (0)...(9)
   <223> Position 7 is D-isomer of lysine
<400> 65
   Gly Leu Val Thr Tyr Lys Lys Ile Lys
   1 5
<210> 66
   <211> 8
   <212> PRT
   <213> Artificial Sequence
<220>
   <223> Lyn
   Position 1 is palmitoyl-glycine
<221> AMIDATION
   <222> (0) ... (8)
<400> 66
   Gly Ile Val Thr Tyr Gly Lys Ile
   1 5
<210> 67
   <211> 8
   <212> PRT
   <213> Artificial Sequence
<220>
   <223> Lyn
   Position 1 is palmitoyl-glycine
<221> AMIDATION
   <222> (0) ... (8)
<221> VARIANT
   <222> 7
   <223> Xaa = Orn
<221> VARIANT
   <222> 7
   <223> Xaa = Any Amino Acid
<400> 67
   Gly Ile Val Ser Tyr Gly Xaa Ile
   1 5
<210> 68
   <211> 8
   <212> PRT
   <213> Artificial Sequence
<220>
   <223> Lyn
   Position 1 is stearyl-glycine
<221> AMIDATION
   <222> (0)...(8)
<400> 68
   Gly Ile Val Thr Tyr Gly Lys Ile
   1 5
<210> 69
   <211> 8
   <212> PRT
   <213> Artificial Sequence
<220>
   <223> Lyn
   Position 1 is stearyl-glycine
<221> AMIDATION
   <222> (0) ... (8)
<400> 69
   Gly Ile Ile Thr Tyr Gly Lys Ile
   1 5
<210> 70
   <211> 8
   <212> PRT
   <213> Artificial Sequence
<220>
   <223> Lyn
   Position 1 is stearyl-glycine
<221> AMIDATION
   <222> (0) ... (8)
<400> 70
   Gly Ile Phe Thr Tyr Gly Lys Ile
   1 5
<210> 71
   <211> 8
   <212> PRT
   <213> Artificial Sequence
<220>
   <223> Lyn
   Position 1 is stearyl-glycine
<221> AMIDATION
   <222> (0) ... (8)
<400> 71
   Gly Ile Leu Thr Tyr Gly Lys Ile
   1 5
<210> 72
   <211> 6
   <212> PRT
   <213> Artificial Sequence
<220>
   <223> Lyn
   Position 1 is stearyl-glycine
   Position 6 is D-isomer of lysine
<221> AMIDATION
   <222> (0) ... (6)
<400> 72
   Gly Ile Val Thr Tyr Lys
   1 5
<210> 73
   <211> 8
   <212> PRT
   <213> Artificial Sequence
<220>
   <223> Lyn
   Position 1 is stearyl-glycine
   Position 6 is D-isomer of lysine
<221> AMIDATION
   <222> (0)...(8)
<400> 73
   Gly Leu Val Thr Tyr Lys Lys Ile
   1 5
<210> 74
   <211> 8
   <212> PRT
   <213> Artificial Sequence
<220>
   <223> Lyn
   Position 1 is stearyl-glycine
   Position 6 is D-isomer of lysine
<221> AMIDATION
   <222> (0) ... (8)
<400> 74
   Gly Ile Val Thr Tyr Lys Lys Ile
   1 5
<210> 75
   <211> 9
   <212> PRT
   <213> Artificial Sequence
<220>
   <223> Myristate at position 1
   <223> Position 5 is 3, 5-diiodo tyrosine
<223> position 6 is D-isomer of lysine
   <223> Dansyl at position 9
<400> 75
   Gly Leu Val Thr Tyr Lys Lys Ile Lys
   1 5
<210> 76
   <211> 25
   <212> PRT
   <213> Artificial Sequence
<400> 76
   Tyr Glu Ile Val Thr Tyr Gly Lys Ile Pro Tyr Pro Gly Arg Thr Asn
   1 5 10 15
   Ala Asp Val Met Thr Ala Leu Ser Gln
   20 25
<210> 77
   <211> 6
   <212> PRT
   <213> Artificial Sequence
<400> 77
   Ile Val Thr Tyr Gly Lys
   1 5
<210> 78
   <211> 19
   <212> PRT
   <213> Artificial Sequence
<400> 78
   Ile Thr Glu Tyr Met Ala Lys Gly Ser Leu Leu Asp Phe Leu Lys Ser
   1 5 10 15
   Asp Glu Gly
<210> 79
   <211> 7
   <212> PRT
   <213> Artificial Sequence
<400> 79
   Ser Leu Leu Asp Phe Leu Lys
   1 5
<210> 80
   <211> 9
   <212> FRT
   <213> Artificial Sequence
<400> 80
   Leu Tyr Ala Val Val Thr Arg Glu Glu
   1 5
<210> 81
   <211> 18
   <212> PRT
   <213> Artificial Sequence
<400> 81
   Ala Asn Leu Met Lys Thr Leu Gln His Asp Lys Leu Val Arg Leu Tyr
   1 5 10 15
   Ala Val
<210> 82
   <211> 30
   <212> DNA
   <213> Artificial Sequence
<220>
   <223> Antisense
<400> 82
   atgggatgta taaaatcaaa agggaaagac 30

## Description

### BACKGROUND OF THE INVENTION

Protein tyrosine kinases are members of the eukaryotic protein kinase superfamily. Enzymes of this class specifically phosphorylate tyrosine residues of intracellular proteins and are important in mediating signal transduction in multicellular organisms. Protein tyrosine kinases occur as membrane-bound receptors, which participate in transmembrane signaling, or as intracellular proteins which take part in signal transduction within the cell, including signal transduction to the nucleus.

As such, phosphorylation of tyrosine by protein tyrosine kinases is an important mechanism for regulating intracellular events in response to environmental changes. A wide variety of cellular events, including cytokine responses, antigen-dependent immune responses, cellular transformation by RNA viruses, oncogenesis, regulation of the cell cycle and modification of cell morphology and phenotype are regulated by protein tyrosine kinases.

Enhanced protein tyrosine kinase activity can lead to persistent stimulation by secreted growth factors, for example, which, in turn, can lead to proliferative diseases such as cancer, to nonmalignant proliferative disease such as arteriosclerosis, psoriasis and to inflammatory response such as septic shock.

Src is among the first protein kinases described whose uncontrolled expression is directly linked to malignant transformation. The Src family contains several members. Some of these members are ubiquitously expressed while others like Lck, Hck, and Lyn have been, until recently, thought to be expressed primarily in cells of the immune system.

Prostate cancer is a malignancy with high incidence in many countries. It is also a cancer with high morbidity and mortality rates and constitutes one of the leading causes of death or disability among males. Its early detection and eradication is aggressively pursued worldwide.

There exists a need for further methods to alleviate and eliminate prostate cancer. In particular, there exists a need for substances which can be administered to individuals who have or are susceptible to developing prostate cancer

### SUMMARY OF THE INVENTION

The present invention is based on the surprising finding that short peptides, corresponding to short sequences from specific regions of Lyn-kinase, or variants of said sequences, were able to reduce growth of prostate cancer cells both *in vivo* and *in vitro.*

The present invention is further based on the surprising finding that said short peptides were capable of inhibiting Lyn-associated signal transduction, thus leading to the understanding that the inhibition of the Lyn-associated signal transduction (hereinafter *"LAST*") leads to the reduction of growth of prostate cancer cells.

Thus, by a first aspect, the present invention concerns compounds for use in a method for the reduction of growth of prostate cancer cells comprising: administering to the cells a compound comprising an amino acid sequence that corresponds to sequences in specific regions of Lyn-kinase (hereinafter: the "*HJ-loop, B4-B5 region*, αD region, *A-region*") or to variants of said sequence.

The reduction of growth of prostate cancer cells can be used for the treatment of prostate cancer.

By a second aspect, the present invention concerns a use for reducing growth of prostate cancer cells by administration to the cells of at least one inhibitor of *LAST*.

The method may be used as a therapeutic method for the treatment of prostate cancer.

The inhibitors of LAST are compounds having amino acid sequences corresponding to sequences present in the above specific regions of then Lyn- kinase, or variants of said sequences.

Without wishing to be bound by theory, it is assumed that the amino acid sequence, present in said compounds, mimics these specific regions in the Lyn-kinase, which are regions that interact with other cellular components, such as with the substrates of the Lyn-kinase, phosphatases of the kinase, or other kinases that de-phosphorylate, or phosphorylate, respectively, the Lyn-kinase. This mimic sequence is assumed to bind to the other cellular components (for excample to the substrates of the Lyn-kinase) and this binding causes the interruption of the interaction of the Lyn-kinase with said cellular components. This interruption causes the inhibition of the signal transduction mediated by the Lyn-kinase, thus leading to the reduction of the growth of prostrate cancer cells.

### GENERAL DESCRIPTION OF THE INVENTION

By one aspect, the present invention concerns a compound for use in a method for the reduction in the growth of prostate cancer cells having a sequence selected from: K055H302 (SEQ ID NO:61) and K055H719 (SEQ ID NO: 75).

The term *"reduction of growth* " refers to a decrease in at least one of the following: number of cells (due to cell death which may be necrotic, apoptotic or a combination of the above) as compared to control; decrease in growth rates of cells, i.e. the to total number of cells may increase but at a lower level or at a lower rate than the increase in control; decrease in the invasiveness of cells (as determined for example by soft agar assay) as compared to control even if their total number has not changed; and progression of non-differentiated cancer cells to a more differentiated phenotype.

Reduction of growth in the contexts of a treated individual is a clinical term refering to at least one of: decrease in tumor size; decrease in rate of tumor growth; stasis of tumor size; decrease in the number of metastasis; decrease in the number of additional metastasis; decrease in invasiveness of the cancer, decrease in the rate of progression of the tumor from one stage to the next, as well as decrease in the angiogenesis induced by the cancer.

The term *"Lyn-kinase"* in reference to specific positions concerns protein tyrosine kinase denoted as EC 2.7.1.12, splice from A-human Accession TVHULY, PID g66782 (NCBI database).

The term "*region*" refers to a sequence in a specific location is the Lyn - kinase that corresponds to the positions selected from: 434 to 458 (termed: *HJ loop*); positions 318-336 (termed: *αD region*); position 305-313 (termed: *B4-B5 region*) and position 291-308 (termed: *A*-region).

The term *"corresponding D-amino acid"* refers to the replacement of the naturally occurring L-configuration of the natural amino acid residue by the D-configuration of the same residue.

The present invention also concerns use of a compound having a sequence selected from: K055H302 (SEQ ID NO:61) and K055H719 (SEQ ID NO:75) for the preparation of a medicament for the treatment of prostate cancer.

The term *"treatment of prostate* cancer" includes at least one of the following: decrease in the rate of growth of the cancer (i.e. the cancer still grows but at a slower rate); cease of prostate cancer growth, i.e., stasis of the prostate cancer tumor occurs, and, in preferred cases, the prostate cancer tumor diminishes or is reduced in size. The term also concerns reduction in the number of metastasis, reduction in the number of new metastasis formed, slowing of the progression of the cancer from one stage to the other and decrease in angiogenesis induced by the cancer. In most preferred cases, the prostate cancer tumor is totally eliminated. This term also concern prevention for prophylactic situations or for those individuals who are susceptible to contracting prostate tumor cancer, the administration of said compounds will reduce the likelihood of the individual contrasting the disease. In preferred situations, the individual to whom the compound is administered does not contract the disease.

The term *"prostate cancer"* in the context of the present invention concerns both hormone responsive, as well as hormone refractory prostate cancer, as well as benign prostate hypertrophic conditions.

The amount of compounds of the invention administered to the individual will depend on the type and severity of the disease (for example, hormone refractory vs. hormone responsive) and on the characteristics of the individual, such as general health, age, body weight and tolerance to drugs as well as on the mode of administration. The skilled artisan will be able to determine appropriate dosages depending on these and other factors. Typically, a therapeutically effective amount of the compound can range from about 1 mg per day to about 1000 mg per day for an adult Preferably, the dosage ranges from about 1 mg per day to about 100 mg per day.

The LAST inhibitors are compounds having sequences as defined in claim 1, derived from regions of the Lyn-kinase which are responsible for interaction with other cellular components, especially with the substrate. As indicated above, it is assumed that peptides mimicking said regions, bind to the cellular components (such as substrates of the Lyn-kinase), and by this interrupt the interaction of the Lyn-kinase and the substrate, leading to inhibition of LAST. present in Lyn-kinase in positions 305-316 (B4-B5 region);
(d) a sequence which is a continuous stretch of at least five amino acids present in Lyn kinase in positions 291-308 (A-region);
(e) a variant of a sequence according to any one of (a) to (d) wherein up to 40% of the amino acid of the native sequence have been replaced with a naturally or non-naturally occurring amino acid or with a peptidomimetic organic moiety; and/or up to 40% of the amino acids have their side chains chemically modified and/or up to 20% of the amino acids have been deleted, provided that at least 50% of the amino acids in the parent sequence of (a) to (d) are maintained unaltered in the variant and provided that the variant maintains the biological activity of the parent sequence of (a) to (d);
(f) a sequence of any one of (a) to (e) wherein at least one of the amino acids is replaced by the corresponding D- amino acid;
(g) a sequence of any one of (a) to (f) wherein at least one of the peptidic backbones has been altered to a non-naturally occurring peptidic , backbone;
(h) a sequence being the sequence of any one of (a) to (g) in reverse order, and
(i) a combination of two or more of the sequences of (a) to (h)

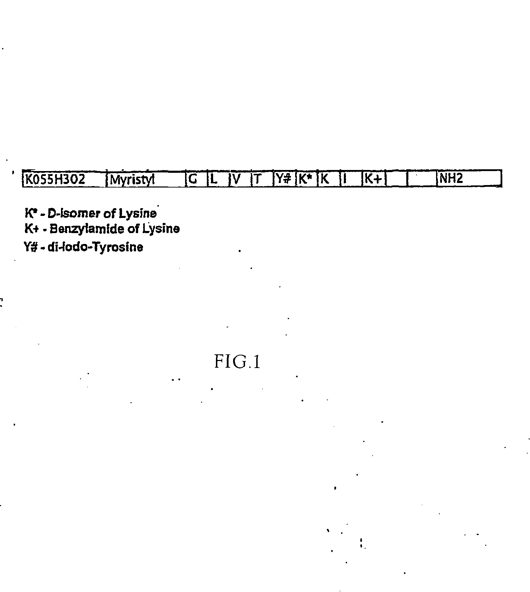

The compounds which have any one of the sequences as specified in SEQ ID NO:61 and SEQ ID NO:75 will be referred to in the following description with the following annotations: K055H302 (SEQ ID NO:61); as specified in Fig 1, and K055H719 (SEQ ID NO:75).

The term *"Lyn-associated signal transduction (LAST)"* refers to the level of signaling mediated by Lyn-kinase, which is best determined by determination of the phosphorylation level of at least one substrate in the lyn-signaling pathway which may be a direct substrate of Lyn (Lyn itself CD19, CD79, Vav, Syk, Shc, PI3-kinase (p85), N-Myristoyltransferase (NMT), FAK, Protein Band 3,Syk,SLP-65, Tec protein tyrosine kinase,HSI) ) or a substrate of another kinase more downstream in the Lyn-kinase signaling pathway, such as MAP kinase, ERK, PKB.

The sequences which correspond to regions of Lyn, in addition to their ability to reduce prostate cancer growth in individuals or their ability to inhibit the growth of prostate cancer cells, also are useful for generating antibodies that reduce prostate cancer growth and inhibit the growth of prostate cancer cells. The sequences act as antigenic agents for producing such antibodies. These antibodies, in turn, act as inhibitors of LAST, thereby reducing prostate cancer growth and inhibiting prostate cancer cell growth when they are administered to the individual with prostate cancer or to the prostate cancer cells.

### BRIEF DESCRIPTION OF THE DRAWINGS

**Fig**. **1** is a Table illustrating the sequence of the peptide K055H302 (SEQ ID NO:61)
**Fig**. **2** is a graph showing the percent inhibition of proliferation of DU145 prostate cancer cells by increasing concentrations of the compounds of the invention K055H302 (SEQ ID NO:61) and K055H719 (SEQ ID NO:75).
**Fig. 3** is a graph showing the percent inhibition of proliferation of PC3 prostate cancer cells by increasing concentrations of the compounds of the invention: K055H302 (SEQ ID NO:61) and K055H719 (SEQ ID NO:75).
**Fig. 4** is a graphical representation of the change of prostate cancer tumor over a period of time for control animals and a group of animals to whom the peptide K055H302 (Bblac) (SEQ ID NO:61) and K055H719 (Bblac) (SEQ ID NO:75) had been administered. **Fig. 4A** - change defined as percentage of change from the initial volume, **Fig.** 4**B** - change defined as absolute change in size of tumor.
**Fig. 5** Western blots showing the phosphorylation levels of several Lyn substrates in the presence and absence of compound of the invention K055H302 (SEQ ID NO:61);
**Fig. 6** Shows soft agar results of the effect of the compound of the invention K055H302 (SEQ ID NO:61), on the invasiveness of DU-145 cells;
**Fig 7** shows co-immunoperciptation results of Lyn and its substrate Syk in the presence and absence of the compound of the invention K055H302 (SEQ ID NO:61).

### DETAILED DESCRIPTION OF THE INVENTION

The present invention is based on the finding that Lyn-tyrosine kinase is active in prostate cells and that the reduction of the signal transduction associated with the kinase (as determined for example by the reduction of the phosphorylation of the kinase substrate) inhibits the growth of prostate cancer cells. Thus the administration of inhibitors of LAST, causes the inhibition of the signal transduction leading to the reduction in prostate cancer growth

This invention is also directed to compounds useful in methods for inhibiting the growth of prostate cancer cells, whether within the body of an individual, in the prostate or in metastasis, or anywhere outside an individual's body, such as in an *in vitro* setting. These methods are directed to administering one or more inhibitors of LAST to the prostate cancer cells. The inhibitor or inhibitors is (are) administered in amounts that are effective in reducing the growth of the prostate cancer cells. When the inhibitors are administered to the prostate cancer cells, the cells stop proliferating (growing or dividing) as rapidly as they did in the absence of the inhibitors. In many instances, growth of the prostate cancer cells entirely ceases for example since the prostate cancer cells lose their viability and die. The growth retardation or death of the prostate cancer cells occurs because Lyn tyrosine kinase associated signal transduction is involved with growth and viability of prostate cancer cells.

Any inhibitor of LAST will thus serve to decrease the level of Lyn-associated signal transduction and thus will act to decrease growth of cancer cells, as will be explained below.

### Compounds comprising Lyn Derived Peptides:

The inventive inhibitors of LAST are compounds which herein are designated as "Lyn-derived peptides". The peptides apparently mimic a region in the kinase and thus bind to other cellular components with which the Lyn-kinase interacts (such as the kinase substrates). This binding interrupts the kinase-component interaction (especially kinase-substrate interaction) and thus inhibit LAST. It should be noted that Lyn-kinase may form dimmers with other Lyn-kinases, leading to trans-phosphorylation so that the substrate may be the Lyn-kinase itself.

This LAST inhibition causes a reduction in the growth of prostate cancer tumors *in vivo.* Quite often the tumors are reduced in size and many times are eliminated altogether.

The peptides according to the above non-limiting theory mimic a region in the Lyn- tyrosine kinase that is involved in the interaction of the Lyn-tyrosine kinase with other cellular components that are part of the Lyn-associated signal transduction. Preferably, these cellular components are selected from: the substrates of Lyn-kinase, other kinases (which may be other Lyn-kinase proteases for trans-phosphorylation, or kinases of the same or different family), phosphatases, as well as co-factors and ATP Thus, any peptide which mimics a part of the Lyn-kinase responsible for said interaction can bind to the cellular component, and thus inhibit the LAST.

The finding of such peptide involves routine screening of the Lyn-kinase regions as will be explained below.

Specific preferred regions of the Lyn kinase that the Lyn-derived peptides mimic are: the HJ-loop, αD-loop, A-region, and B4-B5 region, as defined above. It is clear that for interruption of the kinase-cellular component interaction there is no need to obtain a mimic of the full region of the kinase and a mimic of a subsequence may be sufficient to interrupt said interaction. It is further clear that the interruption may be caused by mimicking of any one of several smaller subsequences in the region and there is no necessity to mimic only one subsequence. It is further clear that for mimicking purposes it is not necessarily to obtain a sequence as present in the native kinase and variants of that sequence ,that can faithfully copy the three dimensional structure of the region (when present in the full kinase), as well as copying the chemical characteristics of several of those side chains that bind to the substrate can also be used as mimics for interruption of the interaction. At times such variants may have better mimicking properties than the native sequence as the variation may help stabilize the mimic amino acid in a more favorable conformation.

### 1. Addition of non-peptidic groups to one or to both of the terminals of the Sequences of the invention to produce the compound of the invention comprising a Lyn-derived peptide

The compound of the invention is a linear molecule, and it is possible to place in any of its terminals various functional groups. The purpose of such a functional group may be for the improvement of the LAST inhibition. The functional groups may also serve for the purpose of improving physiological properties of the compound not related directly to LAST inhibition such as: improvement in stability, penetration (through cellular membranes or barriers), tissue localization, efficacy, decreased clearance, decreased toxicity, improved selectivity, improved resistance to repletion by cellular pumps, and the like. For convenience sake the free N-terminal of one of the sequences contained in the compounds of the invention will be termed as the N-terminal of the compound, and the free C-terminal of the sequence will be considered as the C-terminal of the compound (these terms being used for convenience sake). Either the C-terminus or the N-terminus of the sequences, or both, can be linked to a carboxylic acid functional groups or an amine functional group, respectively.

Suitable functional groups are described in Green and Wuts, "Protecting Groups in Organic Synthesis", John Wiley and Sons, Chapters 5 and 7, 1991, the teachings of which are incorporated herein by reference. Preferred protecting groups are those that facilitate transport of the compound attached thereto into a cell, for example, by reducing the hydrophilicity and increasing the lipophilicity of the compounds these being an example for *"a moiety for transport across cellular membranes*".

These moieties can be cleaved *in vivo,* either by hydrolysis or enzymatically, inside the cell. (Ditter et al, ,J. Pharm Sci. 57:783 (1968); Ditter et al., J. Pharm. Sci. 57:828 (1968); Ditter et al., J. Pharm. Sci. 58:557 (1969); King et al., Biochemistry 26:2294 (1987); Lindberg et al., Drug Metabolism and Disposition 17:311 (1989); and Tunek et al., Biochem. Pharm. 37:3867 (1988), Anderson et al., Arch. Biochem. Biophys. 239:538 (1985) and Singhal et al., FASEB J. 1:220 (1987)). Hydroxyl protecting groups include esters, carbonates and carbamate protecting groups. Amine protecting groups include alkoxy and aryloxy carbonyl groups, as described above for N-terminal protecting groups. Carboxylic acid protecting groups include aliphatic, benzylic and aryl esters, as described above for C-terminal protecting groups.

In addition, a modified lysine residue can be added to the C-terminal of the compound to enhance biological activity. Examples of lysine modification include the addition of an aromatic substitute, such as benzoyl benzoic acid, dansyl-lysine various derivatives of benzoic acids (diffuoro-, trifluromehy-, acetamido-, dimethyl-, dimethylamino-, methoxy-) or various derivatives of carboxylic acid (pyrazine-, thiophene-, pyridine-, indole-, naphthalene-, biphenyl,), or an aliphatic group, such as acyl, or a myristic or stearic acid, at the epsilon amino group of the lysine residue.

Examples of N-terminal protecting groups include acyl groups (-CO-R1) and alkoxy carbonyl or aryloxy carbonyl groups (-CO-O-R1), wherein R1 is an aliphatic, substituted aliphatic, benzyl, substituted benzyl, aromatic or a substituted aromatic group. Specific examples of acyl groups include acetyl, (ethyl)-CO-, n-propyl-CO-, iso-propyl-CO-, n-butyl-CO-, sec-butyl-CO-, t-butyl-CO-, hexyl, lauroyl, palmitoyl, myristoyl, stearyl, oleoyl phenyl-CO-, substituted phenyl-CO-, benzyl-CO- and (substituted benzyl)-CO-. Examples of alkoxy carboxyl and aryloxy carbonyl groups include CH3-O-CO-, (ethyl)-O-CO-, n-propyl-O-CO-, iso-propyl-O-CO-, n-butyl-O-CO-, sec-butyl-O-CO-, t-butyl-O-Co-, phenyl-O-CO-, substituted phenyl-O-CO- and benzyl-O-CO-, (substituted benzyl)- O-CO-. Adamantan, naphtalen, myristoleyl, tuluen, biphenyl, cinnamoyl, nitrobenzoy, toluoyl, furoyl, benzoyl, cyclohexane, norbornane, Z-caproic. In order to facilitate the N-acylation, one to four glycine residues can be present in the N-terminus of the molecule.

The carboxyl group at the C-terminus of the compound can be protected, for example, by an amide (i.e., the hydroxyl group at the C-terminus is replaced with -NH₂, -NHR₂ and NR₂R₃) or ester (i.e. the hydroxyl group at the C-terminus is replaced with -OR₂). R₂ and R₃ are independently an aliphatic, substituted aliphatic, benzyl, substituted benzyl, aryl or a substituted aryl group. In addition, taken together with the nitrogen atom, R₂ and R₃ can form a C4 to C8 heterocyclic ring with from about 0-2. additional heteroatoms such as nitrogen, oxygen or sulfur. Examples of suitable heterocyclic rings include piperidinyl, pyrrolidinyl, morpholino, thiomorpholino or piperazinyl Examples of C-tenainal protecting groups include -NH₂, -NHCH₃, -N(CH₃)₂, -NH(ethyl), -N(ethyl)₂, -N(methyl) (ethyl), -NH(benzyl), -N(C1-C4 alkyl)(beniyl). -NH(phenyl), -N(C1-C4 alkyl) (phenyl), -OCH₃. -O-(ethyl), -O-(n-propyl), -O-(n-butyl). -O-(iso-propyl), -O-(sec- butyl), -O-(t-butyl), -O-benzyl and -O-phenyl.

Preferably the compounds includes in the N-terminal a hydrocarbon having a length of C₄-C₂₀ preferably C₆-C₁₈, most preferably C₈-C₁₆. Example of hydrophobic moieties are: aaystyl, stearyl, lauroyl, palmitoyl and acetyl etc. Other examoles are gernyl-gernyl,acetyl.

### 2. Chemical Modifications

In the present invention the side amino acid residues appearing in the native sequence may be chemically modified, i.e. changed by addition of functional groups. The modification may be in the process of Lyn-synthesis of the molecule, i.e. during elongation of the amino acid chain and amino acid, i.e. a chemically modified amino acid is added. However, chemical modification of an amino acid when it is present in the molecule or sequence ("*in situ*" modification) is also possible.

### 3. Pharmaceutical compositions and therapeutical methods of treatment

The inhibitor of LAST of the present invention or the compounds for reduction of growth of prostate cancer cells can be used as active ingredients (together with a pharmaceutically acceptable carrier) to produce a pharmaceutical composition. The pharmaceutical composition may comprise one, or a mixture of two or more of the different LAST inhibitors of the invention in an acceptable carrier.

The pharmaceutical composition should be used for the treatment of a prostate cancer (hormone responsive or hormone refractory) as well as for the treatment of benign prostate hypertrophy.

The LAST inhibitors of the present invention can be administered parenterally. Parenteral administration can include, for example, systemic administration, such as by intramuscular, intravenous, subcutaneous, or intraperitoneal injection. Compounds which resist proteolysis can be administered orally, for example, in capsules, suspensions or tablets. The compound can also be administered by inhalation or insufflations or via a nasal spray.

The LAST inhibitors can be administered to the individual in conjunction with an acceptable pharmaceutical carrier as part of a pharmaceutical composition for treating the diseases discussed above. Suitable pharmaceutical carriers may contain inert ingredients which do not interact with the compounds. Standard pharmaceutical formulation techniques may be employed such as those described in Remington's Pharmaceutical Sciences, Mack Publishing Company, Easton, PA. Suitable pharmaceutical carriers for parenteral administration include, for example, sterile water, physiological saline, bacteriostatic saline (saline containing about 0.9% mg/ml benzyl alcohol), phosphate-buffered saline, Hank's solution, Ringer's-lactate and the like. Methods for encapsulating compositions (such as in a coating of hard gelatin or cyclodextran) are known in the art (Baker, et al., Controlled Release of Biological Active Agents, John Wiley and Sons, 1986). The formation may be also resources for administration to bone, or in the form of salve, solution, ointment, etc. for topical administration.

The pharmaceutical compositions may also be administered in conjunction with other modes of therapy (chemotherapy, radiotherapy) routinely used in the treatment of prostate cancer.

A "*therapeutically effective amount*" is the quantity of compound which results in an improved clinical outcome as a result of the treatment compared with a typical clinical outcome in the absence of the treatment. *An "improved clinical outcome*" results in the individual with the disease experiencing fewer symptoms or complications of the disease, including a longer life expectancy, as a result of the treatment. With respect to cancer, an *"improved clinical outcome"* includes a longer life expectancy. It can also include slowing or arresting the rate of growth of a tumor, causing a shrinkage in the size of the tumor, a decreased rate of metastasis and/or improved quality of life (e.g., a decrease in physical discomfort or an increase in mobility).

### 4. Determination of LAST inhibiting activity

Some of the conservative substitutions in the essential positions may diminish the inhibiting, while other such conservative substitution in the essential positions may improve these inhibiting activities. The same is true also for deletions, substitutions (both conservative and non-conservative) in non-essential positions, as well as to chemical modifications (in any position) or insertions. In addition the type and size of the non-amino acid portion of the compounds, such as a hydrophobic moiety in one of its terminals may diminish or increase the LAST inhibiting activities. The LAST inhibiting activities that can be determined for example by using one of the assays stipulated below.

### 4.1 Cellular Assays

It can be readily determined whether a compound modulates the activity of a LAST by incubating the compound with cells which have one or more cellular activities controlled by the LAST. Examples of these cellular activities include cell proliferation, cell differentiation, cell morphology, cell survival or apoptosis, cell response to external stimuli, gene expression, lipid metabolism, glycogen or glucose metabolism and mitosis. The cells are incubated with the candidate compound to produce a test mixture under conditions suitable for assessing the level of the LAST. The activity of the LAST is assessed and compared with a suitable control, e.g., the activity of the same cells incubated under the same conditions in the absence of the candidate compound (or in the presence of a control compound). A lesser activity of LAST in the test mixture compared with the control indicates that the candidate compound inhibits LAST.

Suitable cells for the assay include normal cells which express the Lyn-kinase (such as B-cells), cells which have been genetically engineered to express a Lyn-kinase, malignant cells expressing a Lyn-kinase or immortalized cells that express the kinase.

Conditions suitable for assessing activity include conditions suitable for assessing a cellular activity or function under control of the LAST pathway. Generally, a cellular activity or function can be assessed when the cells are exposed to conditions suitable for cell growth, including a suitable temperature (for example, between about 30°C to about 42°C) and the presence of the suitable concentrations of nutrients in the medium (e.g., amino acids, vitamins, growth factors or of specific activators such as cytokines, hormones and the like).

For example, the proliferation of prostate cancer cells may be determined as in Example 2 below, i.e., determination of proliferation (for example as determined by methylene-blue dye assay) of prostate cancer cell lines such as DU-145 and PC3.

Another cellular assay is for determining the change of invasiveness of prostate cancer cells (such as DU-145 and PC-3) by using a soft agar assay, as specified in Example 4 below.

### 4.2 Phosphorylation of substrates (in cellular or cell free assays)

It is possible to assess the LAST activity and the changes in this LAST as compared to control, by determining the phosphorylation level of the substrate proteins of the Lyn kinase. Examples of possible Lyn substrates are: (Lyn itself, CD19, CD79, Vav, Syk, She, PI3-kinase (p85), N-Myristoyltransferase (NMT), FAK, Protein Band 3,Syk,SLP-65, Tec protein tyrosine kinase,HSI). Cells known to express the Lyn-kinase such as for example B-lymphocytes are incubated with a candidate compound for inhibiting the LAST and are activated. Then the cells are lysed, the protein content of the cells is obtained and separated on a gel. The substrates can be identified by use of suitable molecular weight markers, or by using suitable antibodies, reactive against Lyn, CD19, CD79, Syk, Vav, PI3 kinase (p85), She, etc. The level of phosphorylation of the substrate may be determined by suing labeled anti-Tyr antibodies. Alternatively, the suitable substrate may be immuno-precipitated using antibodies. The level of substrate phosphorylation in the immuno- precipitate can be determined by using anti-phosphotyrosine antibodies (see Fujimoto et al., Immunity, 13:47-57 (2000)).

By another option, phosphorylation may be determined in a cell-free system by incubating a mixture comprising Lyn-kinases, the substrate of the kinase and candidate molecules for inhibiting LAST in the presence of ATP under conditions enabling phosphorylation. The proteins are then subjected to gel separation, transferred to nitrocellulose where the substrate band is identified by antibody or molecular weight marker followed by immunoblotting by anti-phosphotyrosine antibody. Alternatively it is possible to use [γ - ³²P] ATP and quantify the amount of radioactivity incorporated in the substrate (See Fujimoto et al., The J. of Immunol. 7088-7094 (1999). Assays concerning phosphorylation of substances can be seen in Example 5.

### 4.3. Tissue or in vivo Assay

Suitable assays for determining inhibition of LAST can be by inducing prostate tumor in an experimental animal, by implanting prostate cell lines (for example Du-145, PC-3) in an experimental animal such as nude mice (subcutaneous) and then testing the effect of the candidate compound on one of the following: tumor size (decease in size, stasis or decreased growth rates as compared to control), progression of tumor to advanced stages (determined by histological techniques), survival of animals, spread of metastasis, angiogenesis and the like. Such an assay is shown in Example 3 below.

### 5. Preparation of Antibodies

The Lyn-derived peptides of the present invention can be useful in the preparation of specific antibodies against Lyn tyrosine kinase. Suitable antibodies can be raised against a Lyn peptide by conjugating the peptide to a suitable carrier, such as keyhole limpet hemocyanin or serum albumin; polyclonal and monoclonal antibody production can be performed using any suitable technique. A variety of methods have been described (see e.g., Kohler et al., Nature, 256:495-497 (1975) and Eur: J. Immunol. 6:511-519 (1976); Milstein et al., Nature 266: 550-552 (1977); Koprowski et al., U.S. Patent No. 4,172,124; Harlow, E. and D. Lane, 1988, Antibodies: A Laboratory Manual, (Cold Spring Harbor Laboratory: Cold Spring Harbor, NY); Current Protocols In Molecular Biology, Vol. 2 (Supplement 27, Summer 1994), Ausubel, F.M. et al., Eds., (John Wiley & Sons: New York, NY), Chapter 11, (1991)). Generally, a hybridoma can be produced by fusing a suitable immortal cell line (e.g., a myeloma cell line such as SP2/0) with antibody producing cells. The antibody producing cell, preferably those of the spleen or lymph nodes, can be obtained from animals immunized with the antigen of interest The fused cells (hybridomas) can be isolated using selective culture conditions, and cloned by limiting dilution. Cells which produce antibodies with the desired specificity can be selected by a suitable assay (e.g., ELISA).

The antibodies can be used to determine if an intracellular lyn tyrosine kinase is present in the cytoplasm of the cell. A lysate of the cell is generated (for example, by treating the cells with sodium hydroxide (0.2 N) and sodium dodecyl sulfate (1%) or with a non-ionic detergent like NP-40, centrifugating and separating the supernatant from the pellet), and treated with anti-lyn peptide antibody specific for lyn tyrosine kinase. The lysate is then analyzed, for example, by Western blotting or immunoprecipitation for complexes between lyn tyrosine kinase and antibody. Anti-lyn peptide antibodies can be utilized for the study of the intracellular distribution (compartmentalization) of lyn tyrosine kinase under various physiological conditions via the application of conventional immunocytochemistry such as immunofluorescence, immunoperoxidase technique and immunoelectron microscopy, in conjunction with the specific anti-lyn peptide antibody.

Antibodies reactive with the lyn peptides are also useful to detect and/or quantitate the lyn tyrosine kinase in a sample, or to purify the lyn tyrosine kinase (e.g., by immunoaffinity purification).

The lyn-derived peptides of the present invention can also be used to identify ligands which interact with lyn tyrosine kinase and which inhibit the activity of lyn tyrosine kinase. For example, an affinity column can be prepared to which a lyn peptide is covalently attached, directly or via a linker. This column, in turn, can be utilized for the isolation and identification of specific ligands which bind the lyn peptide and which will also likely bind the lyn tyrosine kinase. The ligand can then be eluted from the column, characterized and tested for its ability to inhibit lyn tyrosine kinase function.

Peptide sequences in the compounds of the present invention may be synthesized by solid phase peptide synthesis (e.g., t-BOC or F-MOC) method, by solution phase synthesis, or by other suitable techniques including combinations of the foregoing methods. The t-BOC and F-MOC methods, which are established and widely used, are described in Merrifield, J. Am. Chem. Soc. 88:2149 (1963); Meienhofer, Hormonal Proteins and Peptides, C.H. Li, Ed., Academic Press, 1983, pp. 48-267; and Barany and Merrifield, in The Peptides, E. Gross and J. Meienhofer, Eds., Academic Press, New York, 1980, pp. 3-285. Methods of solid phase peptide synthesis are described in Merrifield, R.B., Science, 232: 341 (1986); Carpino, L.A. and Han, G.Y, J. Org. Chem., 37: 3404 (1972); and Gauspohl, H. et al., Synthesis, 5:315 (1992)). The teachings of these references are incorporated herein by reference.

### 6. Preparation of the Compounds

Peptide sequences for producing any of the sequence of the compounds of the invention may be synthesized by solid phase peptide synthesis (e.g., t-BOC or F-MOC) method, by solution phase synthesis, or by other suitable techniques including combinations of the foregoing methods. The t-BOC and F-MOC methods, which are established and widely used, are described in Aarifield, J. Am. Chem. Soc., 88:2149 (1963); Meienhofer, Hormonal Proteins and Peptides, C.H. Li, Ed., Academic Press, 1983, pp. 48-267; and Barany and Aarifield, in The Peptides, E. Gross and J. Meienhofer, Eds., Academic Press, New York, 1980, pp. 3-285. Methods of solid phase peptide synthesis are described in Aarifield, R.B., Science, 232:341 (1986); Carpino, L.A. and Han, G.Y., J. Org. Chem., 37:3404 (1972); and Gauspohl, H. et al., Synthesis, 5:315 (1992)). The teachings of these references are incorporated herein by reference.

### Example 1 - Preparation of Compounds comprising Lyn-derived peptides

The compounds of this invention can be synthesized utilizing a 430A Peptide Synthesizer from Applied Biosystems using F-Moc technology according to manufacturer's protocols. Other suitable methodologies for preparing peptides are known to person skilled in the art. See e.g., Merrifield, R.B., Science, 232: 341 (1986); Carpino, L.A., Han, G.Y, J. Org. Chem., 37: 3404 (1972); Gauspohl, H., et al., Synthesis, 5: 315 (1992)). The teachings of which are incorporated herein by reference.

Rink Amide Resin [4(2',4' Dimethoxyphenyl-FMOC amino methyl) phenoxy resin] was used for the synthesis of C-amidated peptides. The alpha-amino group of the amino acid was protected by an FMOC group, which was removed at the beginning of each cycle by a weak base, 20% piperidine in N-methylpyrrolidone (NMP). After deprotection, the resin was washed with NMP to remove the piperidine. *In situ* activation of the amino acid derivative was performed by the FASTMOC Chemistry using HBTU (2(1-benzo- triazolyl-1-yl)-1,1,3,3-tetramethyluronium) dissolved in HOBt (1-hydroxy- benzotriazole) and DMF (dimethylformamide). The amino acid was dissolved in this solution with additional NMP. DIEA (diisopropylethylamine) was added to initiate activation. Alternatively, the activation method of DCC (dicycbohexylcarbodiimide) and HOBL was utilized to form an HOBt active ester. Coupling was performed in NMP. Following acetylation of the N-terminus (optional), TFA (trifluoroacetic acid) cleavage procedure of the peptide from the resin and the side chain protecting groups was applied using 0.75 g crystalline phenol; 0.25 ml EDT (1,2-ethandithiol); 0.5 ml thioanisoie; 0.5 ml D.I. H₂O; 10 ml TFA.

### Example 2 Inhibition of Proliferation of Prostate Cancer Cells In Vitro by Incubation with compounds comprising Lyn-derived peptides

Human prostate cancer cell lines PC3 and DU145 were obtained from the American Type Culture Collection (ATCC No. 1435-CRL and 81-HTB). These cell lines were grown in RPMI 1640 medium supplemented with penicillin (100 U/ml), streptomycin (100 µg/ml), glutamine (2 mM) and 10% endotoxin free bovine cell serum (Hyclone).

A suspension of the cells at 2x10⁴ cells/ml was prepared in the above described culture medium and distributed 0.180 ml per well (about 4000 cells/well) in the wells of 96 well, flat bottom, tissue culture microtiter plates.

A series of compounds stock solutions were prepared by diluting a 10 mM solution of the compound in 100% DMSO with phosphate buffered saline (PBS) containing 0.1% bovine serum albumin (BSA) to a concentration of 400 µM. These solutions were labeled DMSO. In many instances, 40 µl of the 10 compound in DMSO solution was mixed with 160 µl of 2M NH₄HCO₃ and heated for 40 minutes at 100°C. The resultant solution was then diluted to 400 µM in PBS containing 0.1% BSA. These compounds stock solutions were labeled "tbi ". The concentration of compound in each stock solution was adjusted to nine times the desired concentration of the compound in the assay mixture. 0.020 ml of each compound stock solution was added to the corresponding wells about 2 hours after prostate cancer cell addition, with six replicates for each concentration. In addition, PBS containing 0.1% BSA solution with no added compound was used as a control. The plates were incubated for 72-80 hours at 37°C in a 10% CO₂ humidified incubator. This formulation was termed "*tbi*", and served as a vehicle and as control.

The plates were labeled and the medium discarded. The wells were fixed with 4% formaldehyde PBS (PBS buffered with 10% formalin from Fisher Scientific; Catalog No. HC200-1) ( 0.2 ml/well) for at least 30 minutes. The wells were washed one time with borate buffer (0.2 ml/well) (0.1M, pH 8.5). Freshly filtered 1% methylene blue solution (0.60 ml/well) was then added to the wells and incubated for 10 minutes at room temperature. The wells were then washed five times with tap water, after which the wells were dried completely. 0.20 ml/well of 0.1 N HCl was added to extract the color. After overnight extraction, the O.D, was read at 630 nm to determine the number of cells per well. The procedure for counting cells is described in greater detail in Oliver et al. J. Cell Sci., 92: 513 (1989), the teachings of which are incorporated herein by reference.

The results are shown in Figs 2 and 3. The data in these figures show that two different compounds, comprising two different Lyn derived peptides K055H302 (SEQ ID NO:61); and K055H719 (SEQ ID NO:75), were able to inhibit growth of two different lines of prostate cancer cells PC-3 and DU-145.

### Example 3 Preparation of B-blac formulation

15 mg of the compound were dissolved in 0.25 ml of 4% benzyl alcohol, 4% Pluronic L44 (BASF, Mount Olive, New Jersey) and 2% benzyl benzoate in propylene glycol. To this, 0.125 ml of 2.2% glycine in DDW and 0.125 ml of 50 mM sodium bicarbonate were added while vigorously stirring the tube. The preparation was heated to 100° C for 15min., then homogenized with Polytron (Kinematica, Luzan, Switzerland) for 2' during which 0.5 ml of 03 M lactose were gradually added.

The sequence of heating and homogenizing was repeated once again and after that the preparation was sterilized by heating to 100° C for 30 min.

### Example 4 Prostate Cancer Tumor Shrinkage in Nude Mice

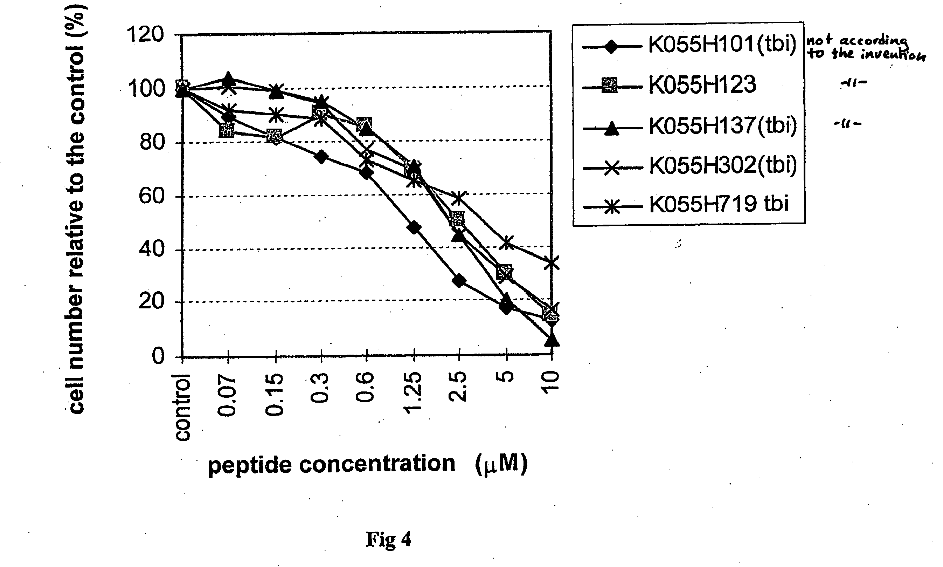

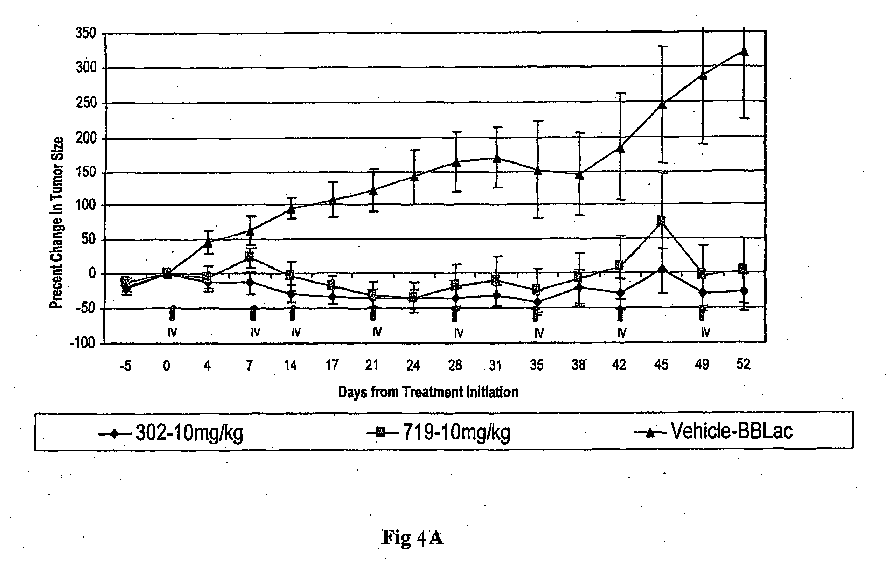

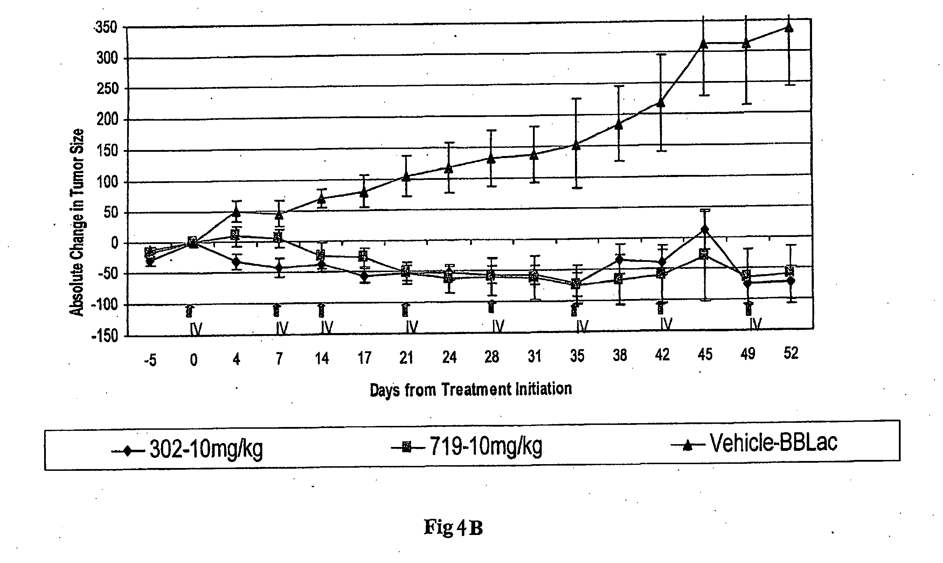

The hormone-refractory human prostate cancer cell line, DU-145, was grown in RPMI-1640 culture medium with 10% fatal calf serum plus penicillin (100 U/ml), streptomycin (100 µg/ml, glutamine (2 mM) (see Example 2). The DU-145 cells were harvested and injected subcutaneously into male nude mice strain CD1 of about 6-7 weeks of age, 5x10⁶ cells per mouse. After about 6 to 8 weeks, when the tumors became palpable, treatment of these mice was started by i.v. injection of 10 mg/kg of a solution comprising either compound K055H302 (SEQ ID NO:61) or K055H719 (SEQ ID NO:75). The compound solutions were prepared by taking BBlac formulation (see example 3) and diluting it 1:8 with lactose (0.3M). Mice received 0.2ml of this solution. Control mice received i.v. injections of vehicle only. Tumor volume was measured twice a week. The results in Fig. 4A shows the change in tumor size, in percentage, from initial tumor, averaged for each group. The results in Fig. 4B show the absolute change in tumor size as compared to control averaged for each group.

As can be seen the tumor diminishes in size with time when compound injections are administered. By contrast, the tumors in control animals grow exponentially over the same time period. Clearly, the compound had a very significant effect on the decrease of the size prostate cancer . In some animals tumor was completely eliminated.

### Example 5 Change of phosphorylation of substrates.

Experimental: cell lymphocytes cell line WEHI-231 was used. 5x10⁶ WEHI-231 cells/sample were washed with serum-free RPMI media (cells were spun at 1700 rpm for 5 min. at 4°C). The cells were suspended in serum-free RPMI media at 2x10⁷ cells/ml, and lysed by addition of an equal volume cold 2xLB (80 mM Tris pH 7.5, 2% NP-40, 1% DOC, 0.2 SDS, 50 mM NaPPi, 100 mM NaF, 2 mM Na₃VO₄, protease inhibitor mix) on ice for 15 min. The resulting mixture was spun for 20 min. 17,000 rpm at 4°C and supernatantly the cell extract was saved.

Immuno-precipitation (IP) of each target-protein was done in one batch: to the cell extract 2µg of appropriate Ab/reaction were added and then cells were rotated o/n on at 4°C. 30 µl 50% of slurry sample of protein A/G beads (prewashed 3 times with cold 1xLB) were again added for 3 hr at 4°C. The IP complex was washed (x2) with cold 1xLB and (x2) with cold reaction buffer (50 mM Tris pH 7.5, 10 mM MgCl₂, 0.1 mM Na₃VO₄, 1 mM DTT). The resulting mixture was spun for 1 min. 14,000 rpm at 4°C and each IP batch was divided into separate tubes.

For the kinase assay: appropriate volumes of the compound of K055H302 (SEQ ID NO:61) was added, or a control compound comprising an irrelevant sequence (obtained from a different kinase GRK) or a control of vehicle alone were added to each sample, and incubated for 20 min. at 30°C. Then 10 µM ATP and 5 units exogenous Lyn were added and incubated for 20 min. at 30°C. The reaction was stopped by addition of 8 µl 5x SDS sample buffer and boiled for 5 min. at 100°C. The resulting samples were separated on SDS-PAGE and blotted.

Western blot analysis was carried out with anti-phosphotyrosine, followed by stripping and rehybridization with the relevant antibodies.

The antibodies used in various assays:
- A-pTyr:: Upstate Biotechnology catalog # 05-321
- A-CD19:: Pharmingen catalog # 09651D
- A-Lyn:: Santa Cruz sc-15 (44)
- A-Syk:: Santa cruz sc-1077 (N-19)
- A-Vav:: Santa Cruz sc-132 (C-14).

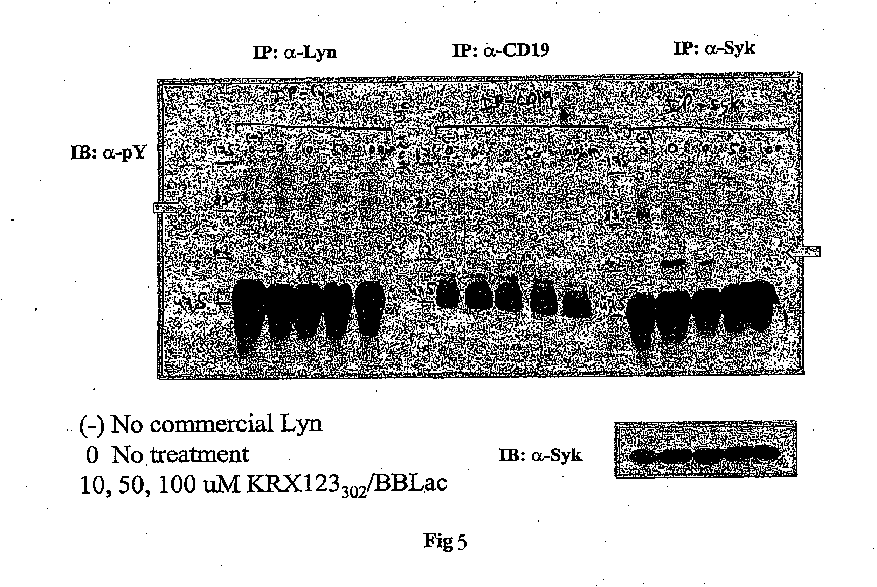

The results are shown in Fig. 5. These results show blots for three immunoprecipitates which are all substrates of Lyn: Lyn itself, CD19 and Syk and the level of phosphorylation is indicated in the absence of Lyn (0), and in the presence of Lyn (+) with increasing concentrations (0, 10, 50 and 100 µM) of the compound K055H302 (SEQ ID NO:61) in B-blac(see example 3). Phosphorylation level was determined with anti-phosphotyrosine.

As can be seen Lyn, CD19 and Syk all showed dose-dependent decreased phosphorylation in the presence of the compound of the invention, thus indicating that the compound is a true LAST inhibitor, as evident by a decrease in the level of its phosphorylation, and that its effects *in vivo* and *in vitro*, shown in the above examples were through inhibition of LAST.

### Example 6 Soft Agar Assay

Reference: Hansen et al., J. Immunol. Met. 119:203 (1989).
Medium I: 2xRPMI Medium
   20% Fetal Calf Serum (FCS)
   4 mM Glutamine
   Den/Strep
Medium II: dilution of MediumI in tissue culture H₂O
Sea Plaque Agarose - FMC BioProducts Catal. #50102
Working solution: 2% agarose = 2 grams /100 ml H₂O
   0.6% agarose = 0.6 grams/100 ml H₂O
MTT - sigma Catalog # M-2128
Working solution: 5 mg/ml PBS, store in dark at 4°C.

### Solubilization Buffer

SDS Electrophoresis Grade - Fisher Catalog #BP 166
N.N-Dimethyl-formamide (DMF) -Fisher Catolog # BP 1160
Acetic Acid, Glacial - fisher catalog #A38

### Working solution:

- Dissolve 40 g SDS in 70 ml warm H₂O and 100 ml DMF, stir in low heat
- When SDS is almost solubilized, add 5 ml 80% acetic acid and 5 ml in HCL to solution. Adjust volume to 200 ml.

### Procedure:

1. Melt agarose in a microwave mix 2% agarose 1:1 with Medium I to give 1% agarose in IX medium.
2. Dispense 100 µl into each well of a 96-well tissue culture plate.
3. Allow base layer to solidify at 40°C for 15 minutes.
4. Mix 0.6% agarose 1:1 with Medium 1 containing DU-145 cells (4000 cells/well) in the presence or absence (control) of the compound of SEC ID 61 and plate 50 µl to each well on top of the under layer.
5. Allow the lower to solidify at 4°C for 15 minutes.
6. Add 50 µl of 4 x drug dilution in Medium II on top of the gel.
7. Incubate plate for 7 days at 37°C 5% CO₂.
8. At end point, add 25 µl MTT to each well.
9. Incubate plate at 37°C, 5% CO₂ for 4 hours.
10. After 4 hours, add 100 µl solubilization solution to each well.
11. Let plat sit overnight in a sealed, humidified container to completely solubilized formazan crystals.
12. Read absorbance at 570 nm wavelength with a reference wavelength of 630 nm using a Dynatech ELISA plate reader, Model MR 500.

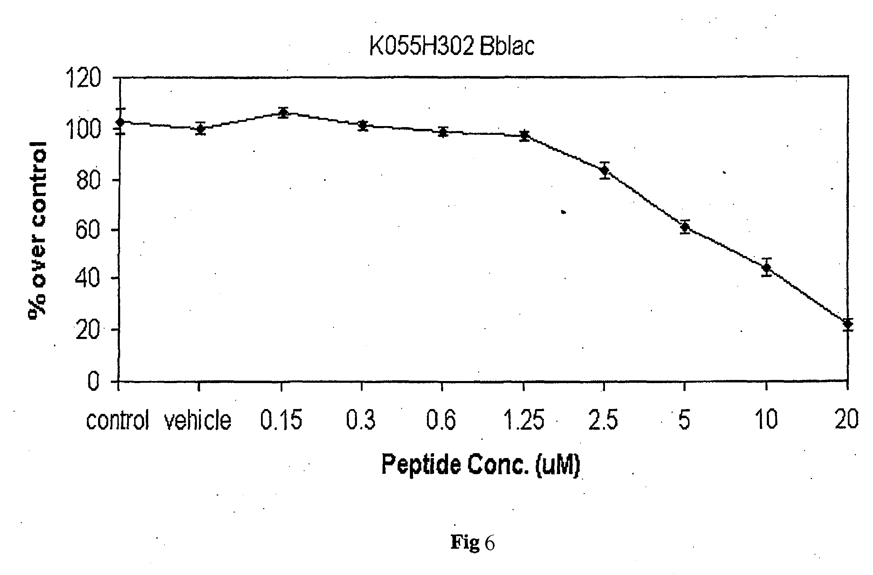

The results are shown in Fig. 6, which show the level of invasivness of DU-145 cells into the soft agar as compared to control in the presence of varying concentrations of the compound of the invention K055H302 (SEQ ID NO: 61). As can be seen a compound comprising an HJ-loop Lyn derived peptide, SEQ Id No 61 was able to reduce the invasiveness of prostate cancer cells DU-145 in a dose dependent manner indicating that the reduction of cancer growth can proceed not only by decrease of proliferation of the cells but also by decrease of their invasiveness.

### Example 7: Interruption Of Interaction Between Lyn And Its Substrate Syk In The Presence Of The Compound Of The Invention.

For proving that thr compound of the invention (SEQ ID NO 61) ,comprising a Lyn derived peptide, blocks the complexation of Lyn with its substrate Syk the amounts of Syk present together with Lyn-kinase, in the presence of varying concentrations of the compound was measured by co-immuno-precipitation (co-IP).

WEHI-231 cells were incubated with 10, 50 and 100 mM of the compound of SEQ ID NO: 61 for 2 hours and following stimulation with a-IgM

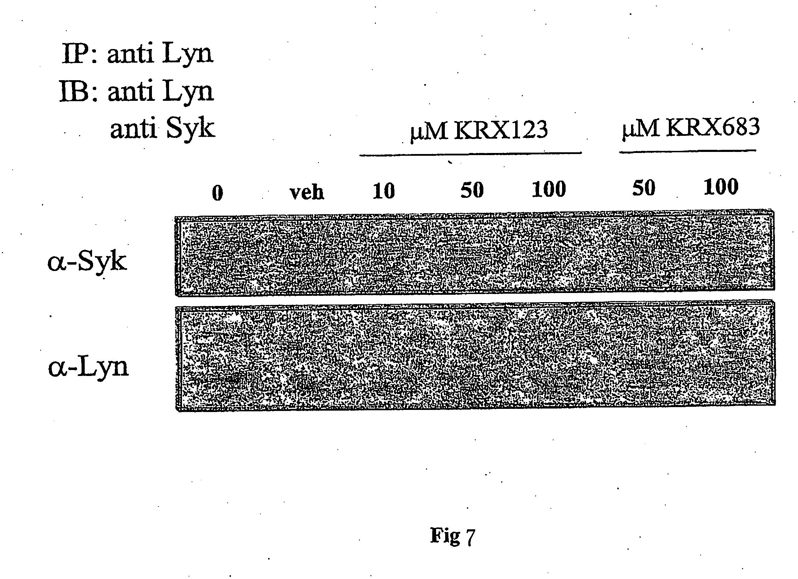

The Lyn was than immunoprecipitated using suitable abti-Lyn antibodies (see procedure of example 5). The Lyn-immunopercipitate was co-immunoreacted with anti-Syk abtibodies. The results are shown in Fig 7. which demonstrates that Syk levels in the Lyn-immunoprecipitates decreased in a dose dependent manner (the amounts of the Lyn itself were not changes). These results support the theory of the invention that the compound comprising the Lyn-derived peptide interrupts the interaction of the Lyn kinase and its substrate (Syk) as can be seen by the decrease in the amount of Syk complexed with the Lyn-kinase. An irrelevant compound comprising a peptide derived from the HJ-loop region of another kinase (GRK ) termed "683" showed no effect.

## Claims

1. A compound selected from K055H302 (SEQ ID NO:61) and K055H719 (SEQ ID NO:75).

2. Use of the compound of claim 1 for the preparation of a medicament for the treatment of prostate cancer.

3. A pharmaceutical composition, comprising the compound of claim 1 and a pharmaceutically acceptable carrier.

4. The compound of claim 1 for use in the treatment of prostate cancer.

## Patentansprüche

1. Verbindung ausgewält aus K055H302 (SEQ ID NO:61) und K055H719 (SEQ ID NO:75).

2. Verwerdung der Verbindung nach Anspruch 1 für die Herstellung eines Medikamentes für die Behandling von Prostatakrebs.

3. Pharmazeutische Zusammensetzung, umfassen die Verbindung nach Anspruch 1 und einen pharmazeutisch verträglichen Träger.

4. Die Verbindung nach Anspruch 1 zur Anwendung für die Behandlung von Prostatakrebs.

## Revendications

1. Composé choisi parmi K055H302 (SEQ ID NO:61) et K055H719 (SEQ ID NO:75).

2. Utilisation du composé selon la revendication 1 pour la préparation d'un médicament destiné au traitement du cancer de la prostate.

3. Composition pharmaceutique comprenant le composé selon la revendication 1 et un véhicule pharmaceutiquement acceptable.

4. Le composé selon la revendication 1 pour utilisation dans le traitement du cancer de la prostate.
